# FASCICULE DE BREVET EUROPEEN

(11) **EP 0 772 630 B1**
(45) Date de publication et mention de la délivrance du brevet: **19.09.2001**
(21) Numéro de dépôt: 95926995.2
(22) Date de dépôt: 31.07.1995
(51) Int. Cl.: C07K 5/12, A61K 38/08

(54) **DERIVES DE STREPTOGRAMINE, LEUR PREPARATION ET LES COMPOSITIONS PHARMACEUTIQUES QUI LES CONTIENNENT**
STREPTOGRAMINDERIVATE, IHRE HERSTELLUNG UND SIE ENTHALTENDE PHARMAZEUTISCHE ZUBEREITUNGEN
STREPTOGRAMINE DERIVATIVES, PREPARATION OF SAME AND PHARMACEUTICAL COMPOSITIONS CONTAINING SAME

(30) Priorité: 02.08.1994 FR 9409563
(43) Date de publication de la demande: 14.05.1997
(73) Titulaire: Aventis Pharma S.A., 92160 Antony (FR)
(72) Inventeur: BARRIERE, Jean-Claude, 91440 Bures-sur-Yvette (FR); PARIS, Jean-Marc, 77360 Vaires-sur-Marne (FR); PUCHAULT, Gérard, 77139 Marcilly (FR)
(86) Numéro de dépôt international: FR9501025
(87) Numéro de publication internationale: WO9604299

(56) Documents cités:
- EP-A- 0 133 096
- BULLETIN DE LA SOCIETE CHIMIQUE DE FRANCE, no. 2, pages 585-591, J. PREUD'HOMME ET AL. 'Pristinamycine isolement, caractérisation et identification des constituants' cité dans la demande

## Description

La présente invention concerne des dérivés de streptogramine de formule générale : 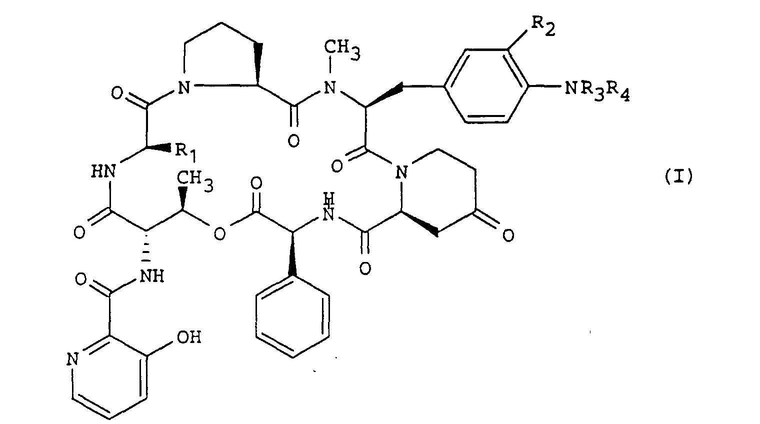 dans laquelle
- le radical R₁ représente un radical méthyle ou éthyle,
- le radical R₂ représente un atome de chlore ou de brome, ou représente un radical alcényle contenant 3 à 5 atomes de carbone si R₃ et R₄ sont des radicaux méthyle et
- les symboles R₃ et R₄ sont l'un un atome d'hydrogène ou un radical méthyle et l'autre un radical méthyle.

Des dérivés solubles appartenant au groupe B des streptogramines ont été décrits précédemment dans les demandes de brevet européens EP 133 097 et EP 248 703. Cependant ces dérivés seuls ou associés à une composante synergisante du groupe A, sont seulement actifs par voie injectable et ne sont pas ou peu actifs par voie orale.

Les dérivés de formule générale (I) définis ci-dessus ouvrent ainsi la voie à de nouvelles streptogramines destinées à un traitement oral. Selon l'invention, les streptogramines de formule générale (I) pour lesquelles R₂ est un atome de chlore ou de brome peuvent être obtenus par action du dérivé N-halogéno succinimide correspondant sur la pristinamycine I pour laquelle R₂ est un atome d'hydrogène.

La réaction s'effectue au moyen de N-chloro ou de N-bromo succinimide dans un solvant organique comme par exemple un solvant chloré (dichlorométhane, dichloréthane, chloroforme) ou un nitrile (acétonitrile), à une température comprise entre 20 et la température de reflux du solvant utilisé.

Selon l'invention, les streptogramines de formule générale (I) pour lesquelles R₂ est un radical alcényle contenant 3 à 5 atomes de carbone peuvent être obtenues par réarrangement en milieu légèrement basique d'un sel dérivé de 4-N-alcénylammonio pristinamycine IA de formule générale : 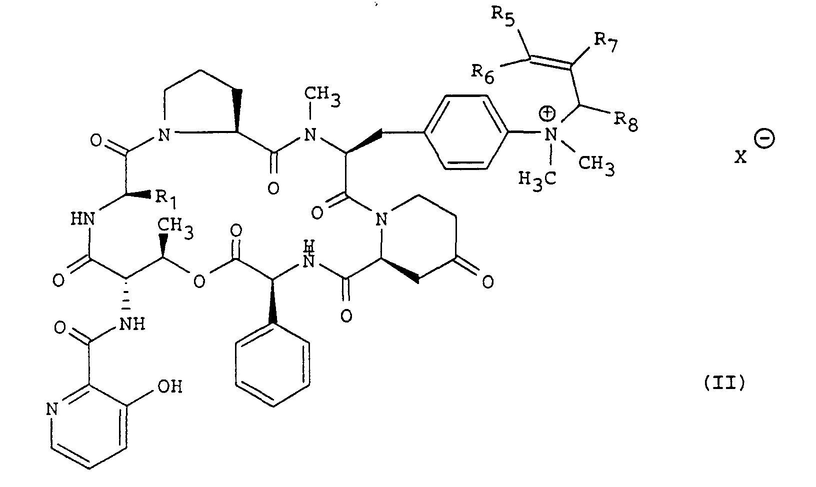 dans laquelle R₁ est défini comme ci-dessus, R₅, R₆, R₇ et R₈ sont un atome d'hydrogène ou un radical méthyle, pourvu que 2 d'entre eux au moins soient des atomes d'hydrogène et X^{⊖} représente un anion, pour donner le dérivé de formule générale : 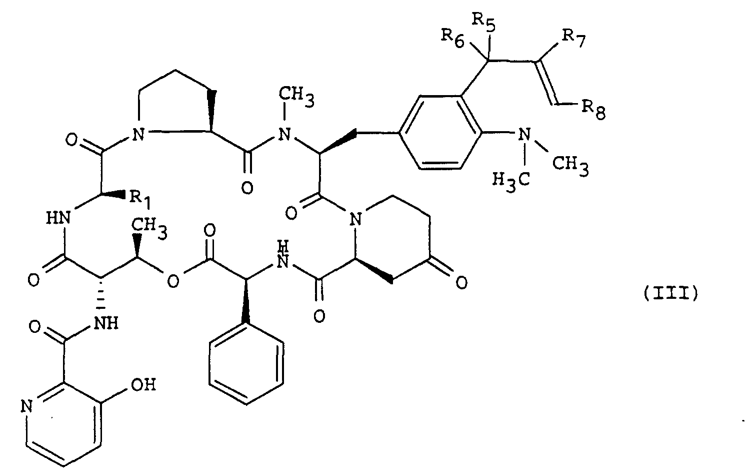 pour lequel R₁, R₅, R₆, R₇ et R₈ sont définis comme ci-dessus.

La réaction s'effectue par chauffage à une température comprise entre 80 et 100°C en milieu aqueux ou biphasique (par exemple en milieu acétate d'éthyle/eau), en présence d'acétate de sodium ou de bicarbonate de sodium ou de potassium. On utilise avantageusement un halogénure de 4-N-alcénylammonio pristinamycine IA.

L'halogénure de 4-N-alcénylammonio pristinamycine IA peut être obtenu par action d'un halogénure d'alcényle de formule générale : 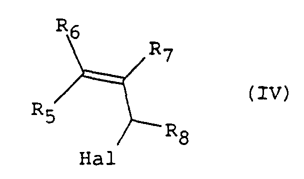 pour lequel R₅, R₆, R₇ et R₈ sont définis comme ci-dessus, et Hal représente un atome d'halogène, sur un dérivé de la pristinamycine de formule générale : 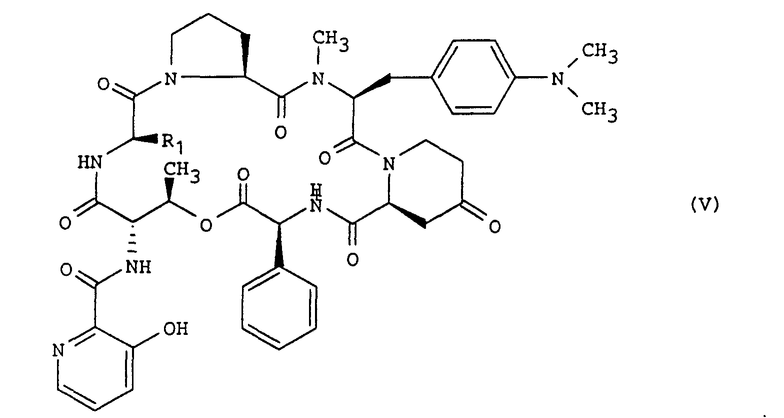 dans laquelle R₁ est défini comme précédemment.

La réaction s'effectue avantageusement dans un solvant organique tel qu'un solvant chloré (dichlorométhane, dichloréthane, chloroforme par exemple) ou un alcool (éthanol par exemple) ou dans un mélange, à une température comprise entre 20°C et la température de reflux du mélange réactionnel. De préférence on fait agir un produit de formule générale (IV) pour lequel Hal est un atome de chlore ou de brome.

Les produits de formule générale (V) sont des produits connus, qui sont décrits par J. Preud'Homme, P. Tarridec, et A. Belloc, Bull. Soc. Chim. Fr., 2, 585 (1968).

Les nouveaux dérivés de streptogramine de formule générale (I) peuvent être purifiés le cas échéant par des méthodes physiques telles que la cristallisation ou la chromatographie.

Les dérivés de streptogramine selon la présente invention présentent des propriétés antibactériennes et des propriétés synergisantes de l'activité antibactérienne des dérivés de la pristinamycine II.

In vivo, il a été montré qu'ils synergisent l'activité antimicrobienne de la pristinamycine II_{B} sur les infections expérimentales de la souris à Staphylococcus aureus IP 8203 à des doses comprises entre 30 et 150 mg/kg par voie orale '(association 30/70).

Leur toxicité (DL50) est supérieure à 1000 mg/kg par voie orale.

Les exemples suivants illustrent la préparation des produits selon l'invention.

Dans les exemples qui suivent, les spectres de RMN ont été étudiés dans le deutérochloroforme, la nomenclature utilisée est celle de J.O. Anteunis et coll., Eur. Biochem., 58, 259 (1975) et notamment : 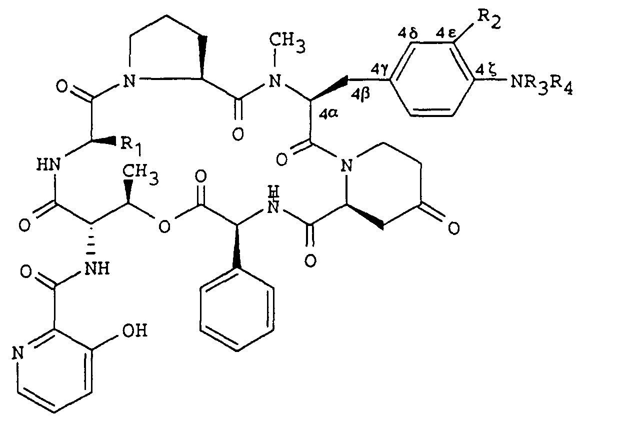 à titre d'exemple les protons en 4δ et 4ε sont respectivement nommés comme H₂, H₃ de l'aromatique en 4 ; les chromatographies flash sont effectuées selon W.C. Still et coll., J. Org. Chem., 43, 2923 (1978), sous une pression d'azote moyenne de 50 kPa en utilisant une silice de granulométrie 40-53µm ; dans tous les cas, le suivi de la chromatographie flash est réalisé par chromatographie sur couche mince.

### Exemple 1

### 4ε-chloro pristinamycine I_{A}.

On place dans un ballon 8 g de pristinamycine IA dans 80 cm³ d'acétonitrile puis on ajoute 1,39 g de N-chlorosuccinimide. Le mélange est chauffé au reflux pendant 16 heures 30 minutes puis on ajoute 0,12 g de N-chlorosuccinimide et on poursuit le reflux 3 heures. Le mélange réactionnel est concentré à sec sous pression réduite (2,7 kPa) à 30°C. Le solide obtenu est repris par 50 cm³ de dichlorométhane et 60 cm³ d'eau distillée additionnée de chlorure de sodium, la phase aqueuse est décantée puis la phase organique lavée par 50 cm³ d'eau distillée saturée en chlorure de sodium. La phase organique est décantée, séchée sur sulfate de magnésium, filtrée puis concentrée à sec sous pression réduite (2,7 kPa) à 30°C pour donner un solide jaune qui est recristallisé dans 100 cm³ de propanol-1 au reflux puis une deuxième fois dans 50 cm³ de propanol-1 au reflux. Après refroidissement, filtration des cristaux et séchage sous pression réduite (135 Pa) à 50°C on obtient 3g de 4ε-chloro pristinamycine I_{A} sous forme de cristaux beige clair fondant à 220°C.

Spectre de R.M.N. du proton (300 MHz, CDCl₃, δ en ppm): 0,58 (dd, J=16 et 6 Hz, 1H, 5 β₂) 0,91 (t, J=7,5 Hz, 3H: CH₃ 2 γ), de 1,05 à 1,35 (mt, 2H: 3 β₂ et 3 γ₂), 1,32 (d, J=7,5 Hz, 3H: CH₃ 1 γ), de 1,50 à 1,85 (mt, 3H: 3 γ₁ et CH₂ 2 β), 2,03 (mt, 1H, 3 β₁), 2,17 (mt, 1H, 5 δ₂), 2,39 (d large, J=16 Hz, 1H: 5 δ₁), 2,44 (d, J=16 Hz, 1H: 5 β₁), 2,77 (s, 6H: N(CH₃ )₂ 4), 2,85 (dt, J=13,5 et 4,5 Hz, 1H: 5 ε₂), 2,97 (dd, J=12 et 5 Hz, 1H: 4 β₂), 3,23 (s, 3H: NCH₃ 4), 3,35 (t, J=12 Hz, 1H: 4 β₁), 3,30 et 3,58 (2 mts, 1H chacun: CH₂ 3 δ), 4,57 (dd, J=8 et 7,5 Hz, 1H, 3 α), 4,76 (dd large, J=13,5 et 8 Hz, 1H: 5 ε₁), 4,85 (mt, 1H: 2α), 4,90 (dd, J=10 et 1,5 Hz, 1H: 1α), 5,25 (dd, J=12 et 5 Hz, 1H: 4 α), 5,31 (d large, J=6 Hz, 1H: 5 α), 5,86 (d, J=9,5 Hz, 1H: 6 α), 5,90 (mt, 1H: 1β), 6,50 (d, J=10 Hz, 1H: NH 2), 6,97 (d, J=8 Hz, 1H: H 5 de l'aromatique en 4), 7,08 (dd, J=8 et2 Hz, 1H: H 6 de l'aromatique en 4), de 7,15 à 7,40 (mt, 6H: H Aromatiques 6 et H 2 de l'aromatique en 4), 7,43 (dd, J=8,5 et 2 Hz, 1H: 1' H₄), 7,52 (dd, J=8,5 et 4,5 Hz, 1H: 1' H₅), 7,83 (dd, J =4,5 et 2 Hz, 1H: 1' H₆), 8,38 (d, J=10 Hz, 1H: NH 1), 8,73 (d, J=9,5 Hz, 1H: NH 6), 11,65 (s, 1H: OH).

### Exemple 2

### 4ε-bromo pristinamycine I_{A}

On place dans un ballon 30 g de pristinamycine I_{A} dans 300 cm³ de dichlorométhane puis on ajoute 6,85 g de N-bromosuccinimide. Le mélange est agité à température ambiante pendant 29 heures puis concentré à sec sous pression réduite. Le solide obtenu est agité dans 400 cm³ d'éther diéthylique, filtré puis lavé par 2 fois 100 cm³ d'éther diéthylique. Après filtration le solide est trituré pendant 45 minutes dans 400 cm³ d'eau distillée, filtré puis lavé par 2 fois 150 cm³ d'eau. Le solide obtenu est séché puis recristallisé dans 1600 cm³ d'éthanol au reflux. Après refroidissement, filtration des cristaux et séchage sous pression réduite (135 Pa) à 50°C on obtient 23,2 g de 4ε-bromo pristinamycine I_{A} sous forme de cristaux blancs fondant à 220°C.

Spectre de R.M.N. du proton (300 MHz, CDCl₃, δ en ppm): 0,58 (dd, J=16 et 6 Hz, 1H, 5 β₂), 0,91 (t, J=7,5 Hz, 3H: CH₃ 2 γ), de 1,10 à 1,40 (mt, 2H: 3 β₂ et 3 γ₂), 1,32 (d, J=7,5 Hz, 3H: CH₃ 1 γ) , de 1,50 à 1,85 (mt, 3H: 3 γ₁ et CH₂ 2 β), 2,03 (mt, 1H, 3 β₁), 2,19 (mt, 1H, 5 δ₂), 2,39 (d large, J=16 Hz, 1H: 5 δ₁), 2,44 (d, J=16 Hz, 1H: 5 β₁), 2,76 (s, 6H: N(CH₃ )₂ 4), 2,83 (dt, J=13,5 et 4 Hz, 1H: 5 ε₂), 2,97 (dd, J=12,5 et 4,5 Hz, 1H: 4 β₂) 3,23 (s, 3H: NCH₃ 4), 3,30 et 3,57 (2 mts, 1H chacun: CH₂ 3 δ), 3,33 (t, J=12,5 Hz, 1H: 4 β₁), 4,55 (dd, J=8 et 7,5 Hz, 1H, 3 α), 4,74 (dd large, J=13,5 et 8 Hz, 1H: 5 ε₁), 4,84 (mt, 1H: 2α), 4,92 (dd, J=10 et 2 Hz, 1H: 1α), 5,27 (dd, J=12,5 et 4,5 Hz, 1H: 4 α), 5,33 (d large, J=6 Hz, 1H: 5 α), 5,88( d, J=9,5 Hz, 1H: 6 α), 5,90 (mt, 1H: 1β), 6,53 (d, J=10 Hz, 1H: NH 2), 7,00 (d, J=8 Hz, 1H: H 5 de l'aromatique en 4), 7,12 (dd, J=8 et 2 Hz, 1H: H 6 de l'aromatique en 4), de 7,15 à 7,40 (mt, 5H: H Aromatiques 6), 7,43 (dd, J=8,5 et 2 Hz, 1H: 1' H₄), 7,46 (d, J=2 Hz, 1H: H 2 de l'aromatique en 4), 7,52 (dd, J=8,5 et 4,5 Hz, 1H: 1' H₅), 7,87 (dd, J =4,5 et 2 Hz, 1H: 1' H₆), 8,41 (d, J=10 Hz, 1H: NH 1), 8,74 (d, J=9,5 Hz, 1H: NH 6), 11,65 (s, 1H: OH).

### Exemple 3

### 4ε-chloro pristinamycine I_{B}

En-opérant comme à l'exemple 1 mais à partir de 1,7 g de pristinamycine I_{B}, de 320 mg de N-chlorosuccinimide dans 17 cm³ d'acétonitrile, on obtient après 1 heure 30 minutes de reflux puis concentration à sec du mélange réactionnel, 1,8 g d'un solide beige qui est purifié par chromatographie flash (éluant dichlorométhane-méthanol, 98/2) pour donner 1,2 g de 4ε-chloro pristinamycine I_{B} sous forme d'un solide jaune pâle fondant à 198°C.

Spectre de R.M.N. du proton (400 MHz, CDCl₃, δ en ppm): 0,79 (dd, J=16 et 5,5 Hz, 1H, 5 β₂), 0,91 (t, J=7,5 Hz, 3H: CH₃ 2 γ), 1,15 (mt, 1H: 3 β₂), de 1,25 à 1,40 (mt, 1H: 3 γ₂), 1,34 (d, J=7,5 Hz, 3H: CH₃ 1 γ), de 1,50 à 1,85 (mt, 3H: 3 γ₁ et CH₂ 2 β), 2,03 (mt, 1H, 3 β₁), 2,23 (mt, 1H, 5 δ₂), 2,40 (d large, J=16 Hz, 1H: 5 δ₁), 2,47(d, J=16 Hz, 1H: 5 β₁), 2,85 (dt, J=13 et 4 Hz, 1H: 5 ε₂), de 2,85 à 2,90 (mt, 1H: 4 β₂) 2,88 (s, 3H: ArNCH₃ 4), 3,25 (s, 3H: NCH₃ 4), 3,28 et 3,58 (2 mts, 1H chacun: CH₂ 3 δ), 3,31 (t, J=12 Hz, 1H: 4 β₁), 4,40 (mf, 1H: ArNH), 4,57 (t, J=7,5 Hz, 1H, 3 α), 4,78 (dd large, J=13 et 8 Hz, 1H: 5 ε₁), 4,84 (mt, 1H: 2α), 4,91 (d large, J=10 Hz, 1H: 1α), 5,23 (dd, J=12 et 5 Hz, 1H: 4 α), 5,36 (d large, J=5,5 Hz, 1H: 5 α), 5,89 (d, J=9,5 Hz, 1H: 6 α), 5,90 (mt, 1H: 1β), 6,51 (d, J=10 Hz, 1H: NH 2), 6,55 (d, J=8 Hz, 1H: H 5 de l'aromatique en 4), 7,0,2 (dd, J=8 et 2 Hz, 1H: H 6 de l'aromatique en 4), 7,13 (d, J=2 Hz, 1H: H 2 de l'aromatique en 4), de 7,15 à 7,40 (mt, 5H: H Aromatiques 6), 7,43 (d large, J=8,5 Hz, 1H: 1' H₄), 7,52 (dd, J=8,5 et 4,5 Hz, 1H: 1' H₅), 7,79 (d large, J =4,5 Hz, 1H: 1' H₆), 8,40 (d, J=10 Hz, 1H: NH 1), 8,75 (d, J=9,5 Hz, 1H: NH 6), 11,63 (s, 1H: OH).

### Exemple 4

### 4ε-bromo pristinamycine I_{B}

En opérant comme à l'exemple 2 mais à partir de 2 g de pristinamycine I_{B}, de 420 mg de N-bromosuccinimide dans 30 cm³ de dichlorométhane, on obtient après 1 heure 30 minutes d'agitation à température ambiante, puis concentration à sec du mélange réactionnel, 2,1 g d'un solide beige qui est purifié par chromatographie flash (éluant dichlorométhane-méthanol, 98/2) pour donner 1,7 g de 4ε-bromo pristinamycine I_{B} sous forme d'un solide blanc fondant à 220°C.

Spectre de R.M.N. du proton (400 MHz, CDCl₃, δ en ppm): 0,80 (dd, J=16 et 5,5 Hz, 1H, 5 β₂) 0,90 (t, J=7,5 Hz, 3H: CH₃ 2 γ), 1,13 (mt, 1H: 3 β₂) de 1,20 à 1,40 (mt, 1H: 3 γ₂), 1,33 (d, J=7,5 Hz, 3H: CH₃ 1 γ), de 1,50 à 1,85 (mt, 3H: 3 γ₁ et CH₂ 2 β), 2,03 (mt, 1H, 3 β₁), 2,28 (mt, 1H, 5 δ₂), 2,40 (d large, J=16 Hz, 1H: 5 δ₁), 2,46 (d, J=16 Hz, 1H: 5 β₁), 2,85 (dt, J=13 et 5 Hz, 1H: 5 ε₂), 2,88 (d, J=5,5 Hz, 3H: ArNCH₃ 4), 2,90 (dd, J=12 et 4 Hz, 1H: 4 β₂), 3,24 (s, 3H: NCH₃ 4), 3,30 et 3,58 (2 mts, 1H chacun: CH₂ 3 δ), 3,31 (t, J=12 Hz, 1H: 4 β₁), 4,41 (q, J=5,5 Hz, 1H: ArNH), 4,57 (t, J=7,5 Hz, 1H, 3 α), 4,78 (dd large, J=13 et 8 Hz, 1H: 5 ε₁), 4,85 (mt, 1H: 2α), 4,91 (d large, J=10 Hz, 1H: 1α), 5,24 (dd, J=12 et 4 Hz, 1H: 4 α), 5,37 (d large, J=5,5 Hz, 1H: 5 α), 5,89 (d, J=9,5 Hz, 1H: 6 α), 5,90 (mt, 1H: 1β), 6,51 (d, J=10 Hz, 1H: NH 2), 6,53 (d, J=8 Hz, 1H: H 5 de l'aromatique en 4), 7,0,5 (dd, J=8 et 2 Hz, 1H: H 6 de l'aromatique en 4), de 7,15 à 7,40 (mt, 6H: H Aromatiques 6 et H 2 de l'aromatique en 4), 7,43 (d large, J=8,5 Hz, 1H: 1' H₄), 7,48 (dd, J=8,5 et 5 Hz, 1H: 1' H₅), 7,79 (d large, J =5 Hz, 1H: 1' H₆), 8,40 (d, J=10 Hz, 1H: NH 1), 8,76 (d, J=9,5 Hz, 1H: NH 6), 11,63 (s, 1H: OH).

### Exemple 5

### 4ε-allyl pristinamycine IA

On place dans un tricol maintenu sous atmosphère d'azote 7,07 g d'acétate de sodium dans 100 cm³ d'eau distillée. La solution est portée au reflux puis on ajoute par une ampoule de coulée, une solution de 15,5 g de bromure de 4-N-allylammonio pristinamycine I_{A} dans 100 cm³ d'eau distillée. Après 2 heures de réaction on ajoute 1 g d'acétate de sodium et le mélange est agité 22 heures au reflux. Une nouvelle portion de 5 g d'acétate de sodium est ajoutée et la réaction poursuivie pendant 20 heures. Le précipité formé est filtré à chaud, rincé par 2 fois 50 cm³ d'eau distillée puis séché sous pression réduite (2,75 kPa) pour donner 7 g d'un solide blanc qui est purifié par chromatographie flash (éluant : toluène, acétone 70/30) pour donner 4,6 g de 4ε-allyl pristinamycine I_{A} sous forme d'un solide blanc fondant à 160°C.

Spectre de R.M.N. du proton (400 MHz, CDCl₃, δ en ppm): 0,42 (dd, J=16 et 5,5 Hz, 1H, 5 β₂) 0,92 (t, J=7,5 Hz, 3H: CH₃ 2 γ), de 1,15 à 1,40 (mt, 2H: 3 β₂ et 3 γ₂), 1,33 (d, J=7,5 Hz, 3H: CH₃ 1 γ), de 1,55 à 1,80 (mt, 3H: 3 γ₁ et CH₂ 2 β), de 2,00 à 2,15 (mt, 2H, 3 β₁ et 5 δ₂), 2,30 (d large, J=16 Hz, 1H: 5 δ₁), 2,33 (d, J=16 Hz, 1H: 5 β₁), 2,63 (s, 6H: N(CH₃ )₂ 4), 2,76 (dt, J=13,5 et 4,5 Hz, 1H: 5 ε₂), 2,98 (dd, J=12 et 4,5 Hz, 1H: 4 β₂) de 3,20 à 3,40 (mt, 3H: 4 β₁ - 3 δ₁ et 1H du ArCH₂ allyle), 3,25 (s, 3H: NCH₃ 4), 3,48 (dd, J=16 et 6,5 Hz, 1H: 1' autre H du ArCH₂ allyle), 3,56 (mt, 1H: 3 δ₂), 4,57 (dd, J=6,5 et 7,5 Hz, 1H, 3 α), 4,68 (dd large, J=13,5 et 7,5 Hz, 1H: 5 ε₁), 4,84 (mt, 1H: 2α), 4,90 (d large, J=10 Hz, 1H: 1α), de 5,00 à 5,15 (mt, 2H: =CH₂), 5,23 (d large, J=5,5 Hz, 1H: 5α), 5,28 (dd, J=12 et 4,5 Hz, 1H 4α), de 5,80 à 5,95 (mt, 3H: 6 α - 1β et CH allyle), 6,53 (d, J=10 Hz, 1H: NH 2), 7,04 (mt, 3H: H Aromatiques en 4), de 7,15 à 7,40 (mt, 5H: H Aromatiques 6), 7,45 (dd, J=8,5 et 2 Hz, 1H: 1' H₄), 7,48 (dd, J=8,5 et 4 Hz, 1H: 1' H₅), 7,88 (dd, J =4 et 2 Hz, 1H: 1' H₆), 8,45 (d, J=10 Hz, 1H: NH 1), 8,76 (d, J=9,5 Hz, 1H: NH 6), 11,64 (s, 1H: OH).

Le bromure de 4-N-allylammonio pristinamycine I_{A} peut être préparé de la manière suivante :

On place dans un tricol maintenu sous atmosphère d'azote, 10 g de pristinamycine I_{A} en solution dans 25 cm³ de dichloro-1,2 éthane puis 2,5 cm³ de bromure d'allyle. Le mélange est chauffé 7 heures à 40°C puis agité à température ambiante pendant 14 heures. On ajoute alors sous agitation en 10 minutes, 200 cm³ de toluène et le mélange est agité 30 minutes. Le précipité formé est filtré, rincé par 50 cm³ de toluène puis séché sous pression réduite (135 Pa) à 45°C pour donner 10,5 g d'un solide qui est trituré dans 200 cm³ d'acétate d'éthyle à 40°C, puis à température ambiante pendant 1 heure. Le solide est filtré puis séché à 45°C sous pression réduite (135 Pa) pour donner 10 g de bromure de 4-N-allylammonio pristinamycine IA sous forme d'un solide blanc fondant vers 210°C.

Spectre de R.M.N. du proton (400 MHz, CDCl₃ avec ajout de quelques gouttes de CD₃ OD d4, δ en ppm): 0,75 (t, J=7,5 Hz, 3H: CH₃ 2 γ), de 1,00 à 1,35 (mt, 3H: 3 β₂ - 3 γ₂ et 5 β₂), 1,18 (d, J=7,5 Hz, 3H: CH₃ 1 γ), de 1,45 à 1,65 (mt, 3H: 3 γ₁ et CH₂ 2 β), 1,95 (mt, 1H: 3 β₁ ), 2,15 (mt, 1H: 5 δ₂), 2,28 (d large, J=16 Hz, 1H: 5 δ₁), 2,55 (d, J=16 Hz, 1H: 5 β₁) 2,72 (dt, J=13,5 et 4,5 Hz, 1H: 5 ε₂), 2,95 (s, 3H: NCH₃ 4), de 3,10 à 3,50 (mt, 4H: CH₂ 4 β et CH₂ 3 δ), 3,40 et 3,48 (2s, 6H en totalité: N(CH₃ )₂ 4), 4,35 (t, J=7,5 Hz, 1H, 3 α), de 4,40 à 4,60 (mt, 3H: NCH₂ allyle et 5 ε₁), 4,64 (mt, 1H: 2α), 4,93 (s large, 1H: 1α), de 5,30 à 5,75 (mt, 7H: CH₂ allyle - 5 α - 4 α-6 α - 1β et CH allyle), 6,88 (d, J=10 Hz, 1H: NH 2), de 7,05 à 7,25 (mt, 8H: H Aromatiques 6 - 1' H₄ et 4 δ), 7,35 (dd, J=8 et 4 Hz, 1H: 1' H₅), 7,60 (d, J=8,5 Hz, 2H: 4 ε), 7,65 (mt, 1H: 1' H₆), 8,58 (d, J=9,5 Hz, 1H: NH 6).

### Exemple 6

### 4ε-(2-méthyl prop-2-ène 1-yl) pristinamycine I_{A}

En opérant comme à l'exemple 5 mais à partir de 4,31 g de chlorure de 4N-(2-méthyl prop-2-ène 1-yl)ammonio pristinamycine I_{A} et de 1,64 g d'acétate de sodium dans 40 cm³ d'eau distillée, on obtient 2,45 g d'un solide qui est purifié par chromatographie flash (éluant toluène, acétone 50/50) pour donner 515 mg de 4ε-(2-méthyl prop-2-ène 1-yl) pristinamycine I_{A} sous forme d'un solide blanc fondant à une température supérieure à 260°C.

Spectre de R.M.N. du proton (400 MHz, CDCl₃, δ en ppm): 0,45 (dd, J=16 et 5,5 Hz, 1H, 5 β₂), 0,90 (t, J=7,5 Hz, 3H: CH₃ 2 γ), de 1,15 à 1,40 (mt, 2H: 3 β₂ et 3 γ₂), 1,33 (d, J=7,5 Hz, 3H: CH₃ 1 γ), de 1,55 à 1,80 (mt, 3H: 3 γ₁) et CH₂ 2 β), 1,66 (s, 3H: CH₃), de 2,00 à 2,15 (mt, 2H, 3 β₁ et 5 δ₂), 2,31 (d très large, J=16 Hz, 2H: 5 δ₁ et 5 β₁), 2,62 (s, 6H: N(CH₃ )₂ 4), 2,78 (dt, J=13 et 4 Hz, 1H: 5 ε₂), 2,99 (dd, J=12 et 3,5 Hz, 1H: 4 β₂), 3,23 et 3,44 (2d, J=15,5 Hz, 1H chacun: ArCH₂), 3,27 (s, 3H: NCH₃ 4), 3,32 et 3,56 (2 mts, 1H chacun: CH₂ 3 δ), 3,33 (t, J=12 Hz, 1H: 4 β₁), 4,58 (t, J=7,5 Hz, 1H, 3 α), 4,60 et 4,82 (2s larges, 1H chacun: =CH₂), 4,70 (dd large, J=13 et 7,5 Hz, 1H: 5 ε₁), 4,84 (mt, 1H: 2α),4,90 (d large, J=10 Hz, 1H: 1α), 5,23 (d large, J=5,5 Hz, 1H: 5α),5,25 (dd, J=12 et 3,5 Hz, 1H: 4α), 5,87 (d, J=9,5 Hz, 1H: 6 α), 5,89 (mt, 1H: 1β), 6,52 (d, J=10 Hz, 1H: NH 2), 7,02 (mt, 3H: H Aromatiques 4), de 7,15 à 7,40 (mt, 5H: H Aromatiques 6), 7,45 (d large, J=8,5 Hz, 1H: 1' H₄), 7,49 (dd, J=8,5 et 4,5 Hz, 1H: 1' H₅), 7,88 (mt, 1H: 1' H₆), 8,45 (d, J=10 Hz, 1H: NH 1), 8,76 (d, J=9,5 Hz, 1H: NH 6), 11,64 (s, 1H: OH).

Le chlorure de 4N-(2-méthyl prop-2ène 1-yl)ammonio pristinamycine I_{A} peut être préparé de la manière suivante :

On place dans un tricol maintenu sous atmosphère d'azote, 8,66 g de pristinamycine I_{A} en solution dans 40 cm³ de dichlorométhane et 20 cm³ de méthanol puis 9,8 cm³ de chlorure de β méthallyle. Le mélange est agité au reflux pendant 48 heures puis concentré sous pression réduite (2,7 kPa) à 30°C. Le solide obtenu est dissous dans 30 cm³ de dichlorométhane puis additionné goutte à goutte sous agitation de 300 cm³ de toluène. Après une heure d'agitation, le solide obtenu est filtré, rincé 3 fois par 30 cm³ de toluène, puis par 50 cm³ d'éther diéthylique. Le solide est filtré puis séché à 45°C sous pression réduite (135 Pa) pour donner 4,34 g de chlorure de 4N-(2-méthyl prop-2ène 1-yl)ammonio pristinamycine I_{A} brut sous forme d'un solide jaune utilisé tel quel pour la préparation de la 4ε-(2-méthyl prop-2-ène 1-yl) pristinamycine I_{A}.

### Exemple 7

### 4ε-[(2-RS)-but-3-ène 2-yl) pristinamycine I_{A} :

En opérant comme à l'exemple 5 mais à partir de 4,8 g de bromure de 4-N-(butène-2-yl) ammonio pristinamycine I_{A}, de 3,69 g d'acétate de sodium dans 100 cm³ d'eau distillée, on obtient 2,37 g d'un solide qui est purifié par chromatographie flash (éluant toluène, acétone 55/45) pour donner 254 mg de 4ε-[(2-RS)-but-3-ène 2-yl) pristinamycine I_{A} sous forme d'un solide blanc fondant à une température supérieure à 260°C.

Spectre de R.M.N. du proton (400 MHz, CDCl₃, δ en ppm) : on observe le mélange des deux diastéréoisomères 50/50. 0,42 et 0,48 (2 dd, J=16 et 5,5 Hz, 1H en totalité, 5 β₂), 0,90 (t, J=7,5 Hz, 3H: CH₃ 2 γ), 1,22 (d, J=7,5 Hz, 3H: CH₃), de 1,15 à 1,40 (mt, 2H: 3 β₂ et 3 γ₂), 1,37 (d, J=7,5 Hz, 3H: CH₃ 1 γ), de 1,55 à 1,80 (mt, 3H: 3 γ₁ et CH₂ 2 β), de 2,00 à 2,15 (mt, 2H, 3 β₁ et 5 δ₂), de 2,15 à 2,40 (mt, 2H: 5 δ₁ et 5 β₁), 2,62 (s, 6H: N(CH₃ )₂ 4), 2,72 et 3,00 (2 mts, 1H en totalité: 5 ε₂), 3,05 et de 3,20 à 3,40 (2 mts, 3H en totalité: 4 β₂ - 4 β₁ et 3 δ₂), 3,27 (s, 3H: NCH₃ 4), 3,57 (mt, 1H: 3 δ₁), 4,10 (mt, 1H: ArCH), 4,60 (t, J=7,5 Hz, 1H, 3 α), 4,64 (dd large, J=13 et 8 Hz, 1H: 5 ε₁), de 4,75 à 5,55 (mt, 6H: =CH₂ - 2α - 1α - 5 α et 4α), de 5,85 à 6,05 (mt, 3H: 6 α - 1β et CH=), de 6,45 à 6,60 (mt, 1H: NH 2), 7,05 (mt, 3H: H Aromatiques 4), de 7,15 à 7,40 (mt, 5H: H Aromatiques 6), 7,45 (mt, 2H: 1' H₄ et 1' H₅), 7,98 et 8,02 (2 mts, 1H en totalité: 1' H₆), 8,53 et 8,57 (2d, J=10 Hz, 1H en totalité: NH 1), 8,82 et 8,85 (2d, J=9,5 Hz, 1H en totalité: NH 6), 11,62 et 11,66 (2s, 1H en totalité: OH).

Le bromure de 4-N-(butène-2-yl) ammonio pristinamycine I_{A} peut être préparé de la manière suivante :

En opérant comme à l'exemple 6 mais à partir de 8,66 g de pristinamycine I_{A}, de 40 cm³ de dichlorométhane, de 20 cm³ de méthanol et de 10,3 cm³ de bromure de crotyle on obtient après 8 heures d'agitation à température ambiante, puis évaporation, un solide qui est dissous dans 40 cm³ de dichlorométhane. A cette solution, on ajoute goutte à goutte sous agitation 400 cm³ de toluène. Après une heure d'agitation, le précipité obtenu est filtré, rincé 3 fois par 30 cm³ de toluène, puis par 50 cm³ d'éther diéthylique. Le solide est filtré pour donner 10,7 g de bromure de 4-N-(butène-2-yl) ammonio pristinamycine I_{A} brut sous forme d'un solide beige clair utilisé tel quel dans la préparation de la 4ε-[(2-RS)-but-3-ène 2-yl) pristinamycine I_{A}.

La présente invention concerne également les médicaments constitués par les dérivés de streptogramines selon l'invention, à l'état pur, associés à un dérivé de pristinamycine II et/ou sous forme d'une association avec tout diluant ou adjuvant compatible et pharmaceutiquement acceptable. Les médicaments selon l'invention peuvent être utilisés par voie orale, rectale ou topique.

Comme compositions pour administration orale peuvent être utilisés des comprimés, des pilules, des poudres ou des granulés. Dans ces compositions le produit actif éventuellement sous forme d'association, est mélangé à un ou plusieurs diluants ou adjuvants inertes, tels que saccharose, lactose ou amidon. Ces compositions peuvent également comprendre des substances autres que des diluants, par exemple un lubrifiant tel que le stéarate de magnésium.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Les compositions pour administration topique peuvent être par exemple des crèmes, des pommades, des lotions ou des aérosols.

En thérapeutique humaine, les nouveaux dérivés de streptogramine selon l'invention sont particulièrement utiles dans le traitement des infections d'origine bactériennes. Les doses dépendent de l'effet recherché et de la durée du traitement. Généralement, les doses sont comprises entre 0,4 et 3,5 g de produit actif en 2 ou 3 prises par jour, par voie orale pour un adulte.

D'une façon générale le médecin déterminera la posologie qu'il estime la plus appropriée en fonction de l'âge, du poids et de tous les autres facteurs propres au sujet à traiter.

L'exemple suivant illustre une composition selon l'invention.

### EXEMPLE

On prépare selon la technique habituelle des comprimés dosés à 250 mg de produit actif, ayant la composition suivante:
- 4ε-allyl pristinamycine I_{A} 250 mg
- pristinamycine II_{B} 75 mg
- excipient : amidon, silice hydratée, dextrine,
   gélatine, stéarate de magnésium : qsp 500 mg

## Revendications

1. Un dérivé de streptogramine de formule générale : 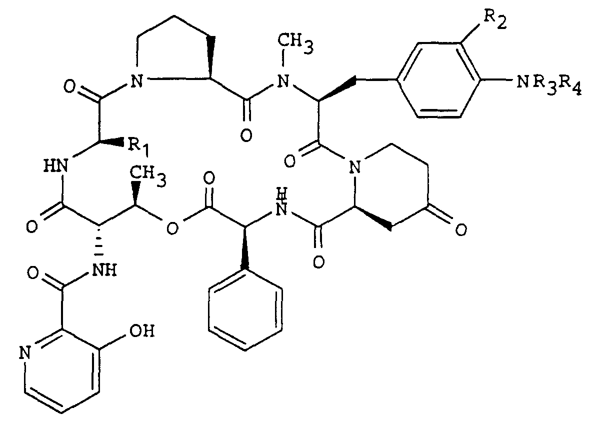 dans laquelle
- le radical R₁ représente un radical méthyle ou éthyle,
- le radical R₂ représente un atome de chlore ou de brome, ou représente un radical alcényle contenant 3 à 5 atomes de carbone si R₃ et R₄ sont des radicaux méthyle et
- les symboles R₃ et R₄ sont l'un un atome d'hydrogène ou un radical méthyle et l'autre un radical méthyle.

2. Procédé de préparation d'un dérivé de streptogramine selon la revendication 1 pour lequel R₂ est un atome de chlore ou de brome, **caractérisé en ce que** l'on fait agir le dérivé N-halogéno succinimide correspondant sur la pristinamycine I pour laquelle R₂ est un atome d'hydrogène.

3. Procédé de préparation d'un dérivé de streptogramine selon la revendication 1 pour lequel R₂ est un radical alcényle contenant 3 à 5 atomes de carbone **caractérisé en ce que** l'on effectue le réarrangement en milieu basique d'un sel dérivé de 4-N-alcénylammonio pristinamycine IA de formule générale : 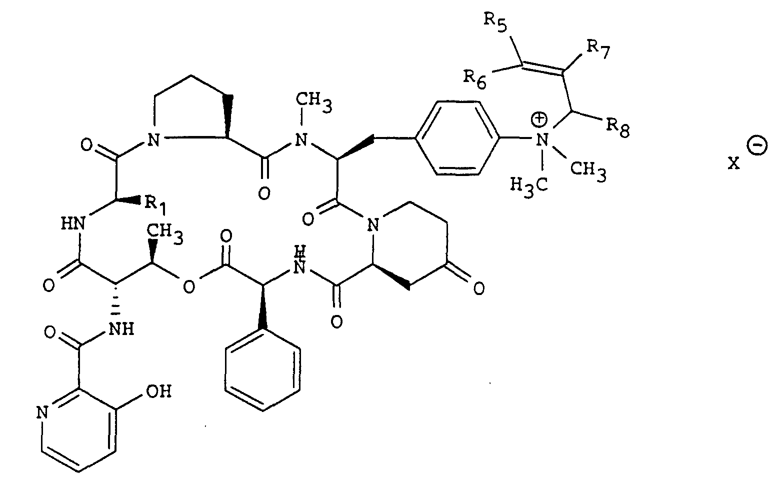 dans laquelle R₁ est défini comme ci-dessus, R₅, R₆, R₇ et R₈ sont un atome d'hydrogène ou un radical méthyle, pourvu que 2 d'entre eux au moins soient des atomes d'hydrogène et X^{⊖} représente un anion.

4. Composition pharmaceutique **caractérisée en ce qu'**elle est constituée d'au moins un dérivé de la streptogramine selon la revendication 1 à l'état pur ou en association avec un dérivé de la pristinamycine II et/ou éventuellement en association avec un ou plusieurs diluants ou adjuvants compatibles et pharmaceutiquement acceptables.

## Claims

1. A streptogramin derivative of general formula : 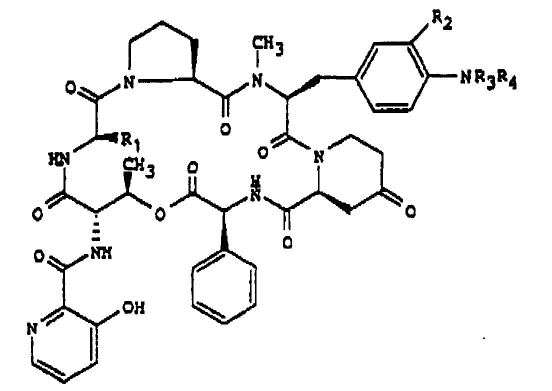 in which
- the radical R₁ denotes a methyl or ethyl radical,
- the radical R₂ denotes a chlorine or bromine atom or denotes an alkenyl radical containing 3 to 5 carbon atoms if R₃ and R₄ are methyl radicals, and
- the symbols R₃ and R₄ are: one, a hydrogen atom or a methyl radical and, the other, a methyl radical.

2. Process for the preparation of a streptogramin derivative according to Claim 1, in the case of which R₂ is a chlorine or bromine atom, **characterized in that** the corresponding N-halosuccinimide derivative is reacted with pristinamycin I in the case of which R₂ is a hydrogen atom.

3. Process for the preparation of a streptogramin derivative according to Claim 1, in the case of which R₃ is an alkenyl radical containing 3 to 5 carbon atoms, **characterized in that** the rearrangement is performed, in basic medium, of a salt derived from 4-N-alkenylammoniopristinamycin IA of general formula: 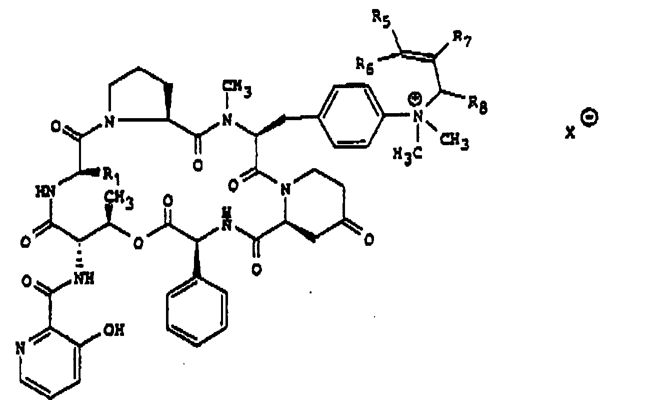 in which R₁ is defined as above, R₅, R₆, R₇ and R₈ are a hydrogen atom or a methyl radical, provided that at least 2 of them are hydrogen atoms, and X⁻ denotes an anion.

4. Pharmaceutical composition **characterized in that** it consists of at least one streptogramin derivative according to Claim 1 in the pure state or in combination with a derivative of pristinamycin II and/or optionally in combination with one or a number of compatible and pharmaceutically acceptable diluents or adjuvants.

## Patentansprüche

1. Streptogramin-Derivat der allgemeinen Formel 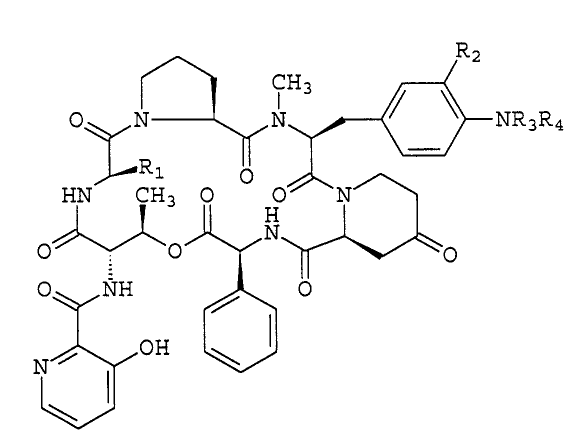 worin
- der Rest R₁ ein Rest Methyl oder Ethyl ist,
- der Rest R₂ ein Chloratom oder ein Bromatom oder einen Rest Alkenyl mit 3 bis 5 Kohlenstoffatomen darstellt, wenn R₃ und R₄ Reste Methyl sind, und
- bei den Symbolen R₃ und R₄ das eine ein Wasserstoffatom oder einen Rest Methyl bedeutet und das andere ein Rest Methyl ist.

2. Verfahren zur Herstellung eines Streptogramin-Derivates nach Anspruch 1, worin R₂ ein Chloratom oder ein Bromatom ist, **dadurch gekennzeichnet, daß** man das entsprechende N-Halogen-succinimid-Derivat mit Pristinamycin I, worin R₂ ein Wasserstoffatom ist, zur Reaktion bringt.

3. Verfahren zur Herstellung eines Streptogramin-Derivates nach Anspruch 1, worin R₂ ein Rest Alkenyl mit 3 bis 5 Kohlenstoffatomen ist, **dadurch gekennzeichnet, daß** man die Umlagerung im basischen Medium eines von 4-N-Alkenylammonio-pristinamycin IA der allgemeinen Formel 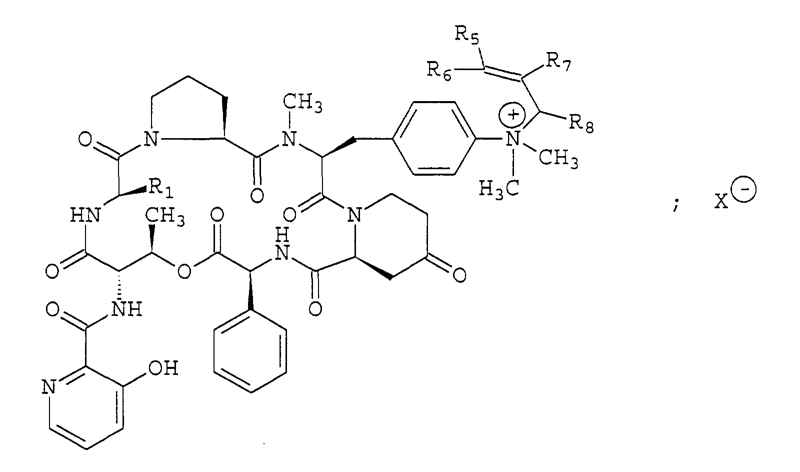 abgeleiteten Salzes durchführt, worin
R₁ wie oben definiert ist, R₅, R₆, R₇ und R₈ Wasserstoffatome oder einen Rest Methyl darstellen, vorausgesetzt, daß mindestens zwei von ihnen Wasserstoffatome sind, und X^{⊖} ein Anion bedeutet.

4. Pharmazeutische Zusammensetzung, **dadurch gekennzeichnet, daß** sie mindestens aus einem Streptogramin-Derivat nach Anspruch 1 in reinem Zustand oder in Assoziation mit einem Derivat von Pristinamycin II und/oder gegebenenfalls in Assoziation mit einem oder mehreren kompatiblen und pharmazeutisch akzeptablen Verdünnungsmitteln oder Zusatzstoffen besteht.
